# EUROPÄISCHE PATENTSCHRIFT

(11) **EP 1 660 886 B1**
(45) Veröffentlichungstag und Bekanntmachung des Hinweises auf die Patenterteilung: **26.11.2008**
(21) Anmeldenummer: 04764199.8
(22) Anmeldetag: 17.08.2004
(51) Int. Cl.: G01N 33/552, G01N 33/554, G01N 33/50

(54) **VORRICHTUNGEN ZUM NACHWEIS BIOCHEMISCHER AKTIVITÄT ENTHALTEND GAP JUNCTIONS**
DEVICE FOR DETECTING BIOCHEMICAL ACTIVITY CONTAINING GAP JUNCTIONS
DISPOSITIFS POUR DETECTER UNE ACTIVITE BIOCHIMIQUE AU MOYEN DE JONCTIONS COMMUNICANTES

(30) Priorität: 26.08.2003 DE 10339597
(43) Veröffentlichungstag der Anmeldung: 31.05.2006
(73) Patentinhaber: Bayer Technology Services GmbH, 51368 Leverkusen (DE)
(72) Erfinder: METHFESSEL, Christoph, 42327 Wuppertal (DE); GOOSSENS, John, 51373 Leverkusen (DE)
(86) Internationale Anmeldenummer: PCT/EP2004/009209
(87) Internationale Veröffentlichungsnummer: WO 2005/022158

(56) Entgegenhaltungen:
- WO-A-02/063298
- WO-A-03/063891
- US-A- 6 149 904
- LOIDL-STAHLHOFEN A ET AL: "SOLID-SUPPORTED BIOMOLECULES ON MODIFIED SILICA SURFACES - A TOOL FOR FAST PHYSICOCHEMICAL CHARACTERIZATION AND HIGH-THROUGHPUT SCREENING" ADVANCED MATERIALS, VCH VERLAGSGESELLSCHAFT, WEINHEIM, DE, Bd. 13, Nr. 23, 3. Dezember 2001 (2001-12-03), Seiten 1829-1834, XP001130104 ISSN: 0935-9648 in der Anmeldung erwähnt
- SACKMANN E E ET AL: "Supported membranes on soft polymer cushions: fabrication, characterization and applications" TRENDS IN BIOTECHNOLOGY, ELSEVIER, AMSTERDAM, NL, Bd. 18, Nr. 2, Februar 2000 (2000-02), Seiten 58-64, XP004187251 ISSN: 0167-7799 in der Anmeldung erwähnt

## Beschreibung

Die Erfindung betrifft Vorrichtungen und Verfahren zum Nachweis von biochemischer Aktivität mit einer Nachweisreaktion, wobei die nachzuweisende biochemische Aktivität räumlich getrennt von der Nachweisreaktion verläuft. Die räumliche Trennung wird durch Lipidmembranen und Gap Junctions erreicht.

### Stand der Technik

### Connexine, Connexone und Gap Junctions

Dem Fachmann bekannt sind biologische Protein-Moleküle, sogenannte Connexine, welche bei der Kommunikation zwischen lebenden Zellen eine besondere Rolle spielen. Mittlerweile werden etwa fünfzehn verschiedene Connexine aufgrund ihrer Aminosäuresequenz unterschieden [1][2]. Connexine kommen in allen Vertebraten vor und werden in der Regel mit Abkürzung wie z.B. Cx26 bezeichnet. Hierbei gibt die Zahl die chromatographische Größe der Connexine in kD an. Bekannt sind bislang Connexine mit einem Molekulargewicht zwischen 26 und 56 kD. Alternativ zu dieser Nomenklatur hat sich eine zweite durchgesetzt, die die Connexine anhand von Strukturmerkmalen in wenigstens 3 Klassen a, b und c einteilt und dann die entsprechenden Connexine in den einzelnen Klassen durchnummeriert.

In der Zellmembran lagern sich jeweils sechs Connexine zu einem Connexon zusammen. Ein Connexon ist eine ringförmige Struktur, die die Zellmembran durchquert und grundsätzlich in der Lage ist, einen sehr weiten, unspezifischen Ionenkanal oder eine wassergefüllte Pore zu bilden. Dabei sind diese Poren aber in der Regel geschlossen, solange sich das Connexon in der Membran einer einzelnen, gesunden Zelle befindet. Berühren sich jedoch zwei Zellen, die in ihrer Membran jeweils miteinander kompatible Connexone aufweisen, dann kommt es zwischen zwei Connexonen der gegenüberliegenden Zellen zur Bildung einer Gap Junction (oder auch elektrische Synapse bezeichnet), der nunmehr die beiden Membranen und den Abstand zwischen den Zellmembranen überbrückt. Die Bildung einer Gap Junction erfolgt bei Kontakt in der Regel in wenigen Minuten. Der ausgebildete Gap Junction Kanal ist eine Struktur aus in der Regel 12 identischen oder verschiedenen Connexinen, bzw. aus zwei Connexonen. Der Kanal besitzt eine ggf. verschließbare zentrale Pore mit einem Durchmesser von etwa 1,5 bis 2 nm. Der wesentliche Unterschied zu anderen Membrankanälen ist, das Gap Junction Kanäle zwei aneinanderliegende Zellmembranen durchziehen und damit nicht eine Verbindung zwischen dem Zellinneren mit dem Außenmedium, sondern eine Verbindung zwischen den intrazellulären Medien der beiden Zellen herstellen.

Dabei ermöglichen Gap Junction Kanäle anorganischen Ionen und kleinen wasserlöslichen Molekülen bis zu einer molekularen Masse von ca. 1000 Dalton den direkten Durchgang von dem Cytoplasma der einen Zelle ins Cytoplasma der anderen Zelle. Damit sind die zwei Zellen sowohl mechanisch, elektrisch als auch metabolisch verbunden. Gap Junction Kanäle gehören zu den epithelialen Zell-Zell-Verbindungen und finden sich in nahezu allen Epithelien und vielen anderen Gewebetypen. In der Regel sind viele Gap Junction Kanäle in Form von Feldern organisiert, wobei diese Strukturen dann als Gap Junction im eigentlichen Sinne bezeichnet werden.

Die Gap Junction Kanäle verbundener Zellen sind in der Regel geöffnet und die Connexine gestreckt. Erleidet eine Zelle einen massiven Calciumeinstrom von außen, etwa durch eine Verletzung, so wird die Verbindung zu benachbarten Zellen unterbrochen indem sich die Connexine allosterisch miteinander verwinden.

Connexine können verfügbar gemacht werden durch Aufreinigen von Zellmembranen aus Zellen, die Connexine enthalten, z. B. Augenlinse, Herzmuskel, glatte Muskulatur, oder Epithelzellen sowie durch gentechnische Expression der Connexine in Bakterien, Hefen oder anderen Zellen. Es ist weiter bekannt, dass Connexine durch Verbindung mit einem Marker, wie zum Beispiel einem fluoreszierenden Proteinfragment, versehen werden können so dass ihr Vorhandensein in einer Zellmembran mit einfachen optischen Verfahren nachweisbar wird [3].

Es sind dem Fachmann Verfahren bekannt, mit denen Connexone in künstliche Membranen oder andere zellfreie Systeme eingebaut werden können [4]. Diese Connexone und Gap Junctions weisen häufig noch die gleichen Eigenschaften - wie z.B. Porengröße, Ionenselektivität, elektrisches Verhalten - auf, wie in ihrer natürlichen Umgebung. Es ist bekannt, dass es auch zwischen zwei Connexonen, die in künstliche Membranen eingelagert sind, bei Kontakt der Membranflächen zur Ausbildung eines funktionsfähigen Gap Junction Kanals kommt [5].

Weiterhin ist bekannt, dass Invertebraten eine funktionell ähnliche Klasse von Membranproteinen aufweisen, die Innexine genannt werden [6]. Die hiermit gebildeten Kanäle besitzen allerdings eine größere Pore, die Molekülen bis zu einem Gewicht von 2000 Dalton den Durchgang ermöglicht. Innexine bilden auch Gap Junctions im Sinne der Erfindung.

Es ist weiterhin bekannt, dass auch in Pflanzen Verbindungen zwischen den Zellen vorkommen, die ähnliche Eigenschaften aufweisen wie Gap Junctions und die als Plasmodesmata bezeichnet werden. Diese überspannen ebenfalls die Zellzwischenwand benachbarter Zellen und ermöglichen ebenfalls einer begrenzten Anzahl von Ionen und kleiner Moleküle die Passage von Zelle zu Zelle. Im Gegensatz zu den Kanälen bei tierischen Lebewesen sind die Plasmodesmata jedoch von der Plasmamembran begrenzt. Auch diese Strukturen werden im Sinne der Erfindung unter dem Oberbegriff Gap Junction zusammengefasst.

### Zelluläre Assays

Bekannt sind weiterhin zelluläre Assays, bei welchen eine bestimmte biochemische Aktivität, die innerhalb einer lebenden Zelle stattfinden, durch geeignete Nachweisreaktionen, welche ebenfalls innerhalb der Zelle stattfinden, nachgewiesen werden. Bekannt sind insbesondere Verfahren, in denen Reaktionsprodukte der biochemischen Aktivität, innerhalb der Zelle mit Indikatormolekülen reagieren, welche dann ein leicht detektierbares Signal, z.B. ein Lichtsignal oder eine Farbänderung, hervorrufen. Ein häufig verwendetes Indikatormolekül ist z.B. das Aequorin [7].

Diesen zellulären Assays ist gemeinsam, dass sie auf die Verwendung von Indikatormolekülen beschränkt sind, die nicht toxisch auf die Zellen wirken und die innerhalb der lebenden Zelle ihre Aufgabe erfüllen können. Anderenfalls würden die Zellen durch die toxischen Indikatormoleküle bereits vor oder auch während des Assays so geschädigt, dass der eigentliche Test gar nicht mehr durchgeführt werden könnte.

### Assays an künstlichen Lipidmembranen

Weiterhin ist bekannt, dass Ionenkanälen, Rezeptoren und andere Targetmoleküle in künstliche Lipidmembranen eingebaut werden, und dort durch geeignete Experimente funktionell untersucht werden können [8]. Interessant sind besonders jene Verfahren, die eine künstliche Lipidmembran durch ein geeignetes Substrat in der Weise zu stabilisieren, dass sie mechanisch fester, länger haltbar, und reproduzierbarer werden [9]. Als Substrate finden hier zum Beispiel Silicagele Verwendung, die ggf. zur Verbesserung der Stabilität und Fluidität der Membran mit einer polymeren Zwischenschicht versehen worden sind [10]. Auch können Bilayer auf dem Substrat mit geeigneten Kettenmolekülen stabilisiert werden ('tethered bilayers') [11].

Im Stand der Technik sind Verfahren enthalten, bei denen Targetmoleküle (z.B. Ionenkanäle, Rezeptoren, Enzyme, etc.) in eine auf einem sphärischen, festen Substrat immobilisierte Lipiddoppelschicht eingebracht werden. Die biochemische Aktivität dieser Targetmoleküle kann dann durch unterhalb der Lipiddoppelschicht befindliche Indikatormoleküle nachgewiesen werden [10]. Das zumeist feste Substrat ist beispielsweise ein Silica-Träger auf welchem eine Lipiddoppelschicht aus Lecitin aufgebracht ist. Eine solche Anordnung ist u.A. kommerziell erhältlich unter dem Handelsnamen Transil^{(R)} der Firma Nimbus Biotechnologie (Leipzig, Germany). Zum Nachweis der biochemischen Aktivität von Targetmolekülen (welche direkt in die Lipiddoppelschicht eingelagert sind) werden hierbei unterhalb der Lipiddoppelschicht immobilisierte Calcium-sensitive oder Phosphat-sensitive Farbstoffe verwendet.

Das vorstehend beschriebene Verfahren hat jedoch den Nachteil, dass für die Untersuchung eines jeden Targetmoleküls die entsprechend beschichteten und mit dem Targetmolekül angereicherten Substrate hergestellt werden müssen. Außerdem kann nicht immer gewährleistet werden, dass die Targetmoleküle in dieser "künstlichen" Umgebung die gleichen biochemischen Aktivitäten entfaltet, wie in der "natürlichen" zellulären Umgebung.

WO 2002/063298 offenbart ein Verfahren zur Identifizierung einer Testverbindung, die in der Lage ist, die Funktion von Gap Junctions zu verändern. In diesem Verfahren wird eine Testverbindung zu einer Assay-Reaktion hinzugefügt. Die Assay-Reaktion umfasst eine Senderzelle und eine Empfängerzelle. Die Senderzelle exprimiert ein erstes Connexin auf der Zelloberfläche, welches ein erstes Connexon bildet und welches eine endogene Messenger-Antwort auf einen exogenen Reiz generieren kann. Die Empfängerzelle exprimiert ein zweites Connexin auf der Zelloberfläche, welches ein zweites Connexon bildet und welches keine endogene Reporter-Antwort auf einen exogenen Reiz generieren kann, aber in der Lage ist, eine Reporter-Antwort auf die endogene Messenger-Antwort der Senderzelle zu generieren. Das erste Connexon und das zweite Connexon bilden eine Gap Junction. Gemäß dem Verfahren wird ein exogener Reiz zur Assay-Reaktion hinzugefügt und die Reporter-Antwort detektiert. Künstliche Membrankörper als Empfängerzelle werden nicht offenbart.

### Beschreibung der Erfindung

Ausgehend von dem oben beschriebenen Stand der Technik stellt sich hier die technische Aufgabe, verbesserte Verfahren und Vorrichtungen bereitzustellen, bei denen einerseits für zelluläre Assays nicht einsetzbare Indikatorsysteme (z.B. toxische Nachweisreagentien) angewendet werden können und andererseits die untersuchten Targetmoleküle in ihrer natürlichen zellulären Umgebung untersucht werden können.

Die technische Aufgabe wird erfindungsgemäß dadurch gelöst, dass Vorrichtungen und Verfahren zur Anwendung kommen, bei denen die zu untersuchende biochemische Aktivität räumlich getrennt stattfindet von der die biochemische Aktivität nachweisenden Reaktion. Dadurch ist es möglich, dass z.B. toxische Nachweisreagentien für den Nachweis der biochemischen Aktivität verwendet werden können, während das Targetmolekül immer noch in seiner natürlichen zellulären Umgebung die nachzuweisende biochemische Aktivität entfaltet. Biochemische Aktivität und Nachweisreaktion sind räumlich entkoppelt.

Die räumliche Trennung wird erfindungsgemäß erreicht durch einen Ort der biochemischen Aktivität und einen Ort der Nachweisreaktion, wobei der Ort der biochemischen Aktivität und der Ort der Nachweisreaktion durch mindestens zwei Lipidmembranen getrennt sind und mit Gap Junctions verbunden sind. Weiterhin ist der Ort der Nachweisreaktion ein künstlich erzeugter Membrankörper. ie Lipidmembranen sind jeweils mit Connexinen oder Innexinen besetzt, sodass sich zwischen den Membranen Gap Junctions ausbilden, die einen Durchtritt von Molekülen oder anderen Signalen zwischen dem Ort der biochemischen Aktivität und dem Ort der Nachweisreaktion ermöglichen.

"Biochemische Aktivität", im Sinne der Erfindung, ist jedwede biochemische Aktivität, die in oder an oder an der Oberfläche von biologischen Systemen (z.B. Zellen) stattfindet, stattfinden kann oder durch das biologische System katalysiert werden kann. Biochemische Aktivitäten sind z.B. durch Proteine katalysierte chemische Reaktionen, Signaltransduktionsvorgänge oder Änderungen der physikalischen oder chemischen Zustandsgrößen (wie z.B. pH Wert, Ionenkonzentration, Metabolitkonzentrationen, etc.). Die Proteine, welche Targetmoleküle darstellen, können gelöst, z.B. frei in Cytoplasma, oder auch membrangebunden, z.B. an der Cytoplasmamembran oder an Organellen, vorliegen.

"Nachweisreaktionen", im Sinne der Erfindung, sind chemische oder biochemische Reaktionen, welche die biochemische Aktivität oder deren Auswirkungen nachweisen können bzw. detektierbar machen. Bevorzugte Nachweisreaktionen sind Farbreaktionen, Fluoreszenz- oder Lumineszenzerscheinungen oder auch komplexe biochemische Reaktionen, die den Nachweis der biochemischen Aktivität erlauben.

"Lipidmembranen", im Sinne der Erfindung, sind Lipidmembranen, wie sie dem Fachmann aus biologischen oder nicht-biologischen Systemen bekannt sind. Lipidmembranen enthalten bevorzugt eine Lipiddoppelschicht, welche den freien Durchtritt von hydrophilen Substanzen verhindert Lipidmembranen im Sinne der Erfindung können Moleküle, z.B. Proteine, eingelagert haben. Lipidmembranen können sphärisch oder auch planar ausgeführt sein. Sie können insbesondere auch auf einer festen oder Gel-artigen Unterlage vorgesehen sein. Eine besondere Ausführungsform der Lipidmembran ist eine Lipidmembran aus Lecitin.

"Gap Junctions", im Sinne der Erfindung, sind Verbindungen zwischen zwei räumlichen Bereichen, die durch Lipidmembranen voneinander getrennt sind. Gap Junctions werden von Proteinen ausgebildet, welche die Lipidmembranen und den Zwischenraum zwischen den Membranen überspannen und so einen Durchgang für Stoffe und für Ladungsaustausch ermöglichen.

"Membrankörper", im Sinne der Erfindung, sind von einer Membran umschossene, mit einer Flüssigkeit gefüllte Volumenelemente. Membrankörper im Sinne der Erfindung sind künstlich erzeugte Membrankörper, bei welchen z.B. eine Lipiddoppelschicht ein begrenztes Volumen eines wässrigen Mediums einschließt (Vesikel). Diese Membrankörper enthalten dann vorzugsweise mindestens eine biologische Komponente, z.B. ein in der Lipiddoppelschicht eingelagertes Polypeptid, ein membranständiges Enzym, einen Ionenkanal oder einen G-Protein gekoppelten Rezeptor.

"Farbstoffe", im Sinne der Erfindung, sind Stoffe, die optisch durch Detektion der von ihnen ausgesandten oder der durch sie nicht absorbierten elektromagnetischen Strahlung nachgewiesen werden können.

"Spannungs-sensitive Indikatoren", im Sinne der Erfindung, sind Stoffe, die in Abhängigkeit einer anliegenden elektrischen Potentialdifferenz oder des vorliegenden elektrischen Potentials derart ihre physikalischen, optischen oder katalytischen Eigenschaften ändern, dass diese ein detektierbares Signal hervorrufen.

Dem Fachmann bekannt sind spannungs-sensitive Indikatoren wie z.B. DIBAC [14].

"pH-Wert-sensitive Indikatoren", im Sinne der Erfindung, sind Stoffe, die in Abhängigkeit des pH-Wertes derart ihre physikalischen, optischen oder katalytischen Eigenschaften ändern, dass diese ein detektierbares Signal hervorrufen. Solche Indikatorfarbstoffe, wie zB Phenolrot, Bromthymolblau, Bromphenolblau u.v.a. sind dem Fachmann zahlreich bekannt.

"Calcium-sensitive Indikatoren", im Sinne der Erfindung, sind Stoffe, die in Anwesenheit von Calcium derart ihre physikalischen, optischen oder katalytischen Eigenschaften ändern, dass diese ein detektierbares Signal hervorrufen. Dem Fachmann bekannte Calcium-sensitive Indikatoren sind z.B. das Aequorin und andere Calcium-sensitive Farbstoffe wie z.B FURA-2.

"Supported Bilayer" sind Membranen, die sich auf der einen Seite in Kontakt mit oder in unmittelbarer Nähe von einem geeigneten festen, porösen oder gel-artigen Material befinden. Dadurch werden sie gegenüber frei tragenden Membranen mechanisch stabiler und belastbarer.

"Wirkstoffe", im Sinne der Erfindung, sind Stoffe, welche die Aktivität von biologischen Molekülen beeinflussen können. Bevorzugte Wirkstoffe, im Sinne der Erfindung, sind solche, die spezifisch die Aktivität von einzelnen biologischen Molekülen oder von Gruppen von biologischen Molekülen beeinflussen. Besonders bevorzugte Wirkstoffe sind solche, welche die Aktivität von Rezeptoren und/oder Ionenkanälen beeinflussen. Ganz besonders bevorzugte Wirkstoffe vermögen bestimmte Krankheitsbilder des Menschen oder von Tieren zu heilen, zu lindern oder zu verhindern.

"Wirkstoff-Screening", in Sinne der Erfindung, ist die zielgerichtete Suche nach chemischen oder biologischen Substanzen, die bei einem bestimmten biologischen System einen bestimmten physiologischen Effekt auslösen. Dieser Effekt ist bevorzugt die Modulation der Aktivität eines Targetmoleküls oder die Heilung, Linderung, oder Verhinderung eines bestimmten Krankheitszustands eines Organismus. Wirkstoff-Screening wird bevorzugt im High-Throughput-Screening (HTS) Format durchgeführt. Hierbei wird eine große Anzahl von chemischen Substanzen während des Screening-Verfahrens mit einem Target in Kontakt gebracht und die Auswirkungen der chemischen Substanzen auf das Target wird ausgewertet.

Die Erfindung betrifft insbesondere:
1. Eine Messanordnung zum Nachweis einer biochemischen Aktivität mit einer Nachweisreaktion für besagte biochemische Aktivität, gekennzeichnet durch einen Ort der biochemischen Aktivität und eine Ort der Nachweisreaktion, wobei der Ort der biochemischen Aktivität und der Ort der Nachweisreaktion durch mindestens zwei Lipidmembranen getrennt sind und mit Gap Junctions verbunden sind und wobei weiterhin der Ort der Nachweisreaktion ein künstlich erzeugter Membrankörper ist.
   Die durch die biochemische Aktivität hervorgerufene Änderung (z.B. eine Änderung der Konzentration einer chemischen Substanz oder eine Spannungsänderung) setzt sich durch besagte Gap Junctions vom Ort der biochemischen Aktivität zum Ort der Nachweisreaktion fort. Dort wird die Änderung durch die Nachweisreaktion ggf. verstärkt bzw. detektierbar gemacht und schließlich detektiert. Die Detektion selbst kann irgendwo erfolgen.
2. Eine Messanordnung nach Punkt 1, wobei besagte biochemische Aktivität in einer lebenden Zelle erfolgt.
   Die verwendeten lebenden Zellen können z.B. Zellen, die aus lebenden Geweben durch Dissoziation isoliert wurden (Primärkulturen) sein. Weiterhin können Zellen, die als etablierte Zelllinien in Kultur gehalten werden, wie CHO-Zellen, HEK-Zellen, NIH3T3-Zellen, HeLa-Zellen aber auch transient transfizierte Zellen oder Primärzellen verwendet werden.
3. Eine Messanordnung nach einem der Punkte 1 bis 2, wobei die Nachweisreaktion eine Reaktion mit einem Farbstoff ist
4. Eine Messanordnung nach einem der Punkte 1 bis 2, wobei die Nachweisreaktion eine Reaktion mit einem Calcium-sensitiven Indikator ist.
5. Eine Messanordnung nach einem der Punkte 1 bis 2, wobei die Nachweisreaktion eine Reaktion mit einem Spannungs-sensitiven Indikator ist.
6. Eine Messanordnung nach einem der Punkte 1 bis 2, wobei die Nachweisreaktion eine Reaktion mit einem pH-Wert-sensitiver Indikator ist.
7. Eine Messanordnung nach einem der Punkte 1 bis 6, wobei eine der Lipidmembranen als supported bilayer ausgeführt ist.
8. Eine Messanordnung nach Punkt 7, wobei die supported bilayer als planare supported bilayer ausgeführt ist.
9. Eine Messanordnung nach Punkt 7, wobei die supported bilayer als sphärische supported bilayer ausgeführt ist.
10. Eine Messanordnung nach Punkt 7, wobei die supported bilayer auf einem sphärischen Silica-Träger aufgebracht ist.
11. Eine Messanordnung nach einem der Punkte 1 bis 6, wobei mindestens eine Lipidmembran das Ende einer Kapillare überspannt. Mit einer solchen Anordnung kann eine Nachweisreaktion in einer Glaskapillare stattfinden, wie sie derzeit z.B. für sogenannte "patch-clamp" Anwendungen verwendet wird [12].
12. Eine Vorrichtung zur Bestimmung von biochemischen Aktivitäten enthaltend eine Vielzahl von Messanordnungen gemäß einem der Punkte 1 bis 11.
13.Ein Verfahren zum Nachweis einer biochemischen Aktivität mit einer Nachweisreaktion, gekennzeichnet durch einen Ort der biochemischen Aktivität und einen Ort der Nachweisreaktion, wobei der Ort der biochemischen Aktivität und der Ort der Nachweisreaktion durch mindestens zwei Lipidmembranen getrennt sind und mit Gap Junctions verbunden sind und wobei weiterhin der Ort der Nachweisreaktion ein künstlich erzeugter Membrankörper ist.
14. Verfahren nach Punkt 13, wobei besagte biochemische Aktivität eine Reaktion auf das Vorhandensein eines Wirkstoffs darstellt.
15. Verwendung einer Messanordnung nach einem der Punkte 1 bis 11 oder einer Vorrichtung nach Punkt 12 zum Wirkstoff-Screening

### Kurze Beschreibung der Abbildungen

Figur 1
   zeigt eine erfindungsgemäße Anordnung zur Bestimmung von biologischer Aktivität mit einem Targetmolekül (gleichzeitig der Ort der biochemischen Aktivität) (1), dem davon räumlich getrennten Ort der Nachweisreaktion (2), einer ersten (3) und einer zweiten (4) Lipidmembran, Connexinen (5) und Gap Junctions (6). Der Ort der biochemischen Reaktion (1) ist räumlich getrennt vom Ort der Nachweisreaktion (2) und dennoch funktionell über Gap Junctions (6) verbunden. Die erste Lipidmembran (3) ist planar ausgeführt.
Figur 2
   zeigt eine erfindungsgemäße Anordnung zur Bestimmung von biologischer Aktivität mit einem Targetmolekül (gleichzeitig der Ort der biochemischen Aktivität) (1), dem davon räumlich getrennten Ort der Nachweisreaktion (2), einer ersten (3) und einer zweiten (4) Lipidmembran, Connexinen (5) und Gap Junctions (6). Die Nachweisreaktion findet unterhalb der Lipidmembran (3) auf einem Partikel (z.B. auf einem Transil^{(R)} Partikel) statt, der einen Kern (7) und eine Lipidmembran (3) aufweist. Es sind zwei über Gap Junctions an das Partikel gekoppelte Membrankörper dargestellt.

### Beispiele

### Beispiel 1: Supported Bilayer Membranen mit eingebauten Connexinen

Es werden Connexine aus der Leber aufgereinigt und renaturiert, wie es in der Literatur beschrieben ist [4]. Die Connexine werden nach dem Verfahren von NIMBUS in supported bilayer Membran auf Transil^{(R)} Partikel eingefügt. Das Verfahren kann natürlich nicht nur mit Connexin Cx32 aus der Leber, sondern sinngemäß auch mit anderen Connexinen wie z.B. Connexin Cx43 aus Herzmuskel, und praktisch allen anderen Connexinen durchgeführt werden sowie mit Proteinen vergleichbarer Struktur aus anderen Organismen, wie z.B. Innexinen aus Invertebraten oder Plasmodesmata aus Pflanzen.

In vielen Fällen ist es wichtig, das Membranpotential einstellen zu können. Deshalb ist es zweckmäßig, nach demselben Verfahren in die Membran zusätzlich einen Kalium-selektiven Ionenkanal einzufügen, der es erlaubt, mit Hilfe der externen Kaliumkonzentration das Membranpotential des Transil^{(R)} Partikeln grob einzustellen. In dieser Weise modifizierte Transil^{(R)} Partikel werden im weiteren als "Connexon-Kaliumkanal Partikel" bezeichnet.

### Beispiel 2: Kopplung von Transil^{(R)} Partikeln an Hepatocyten

Die oben beschriebenen Connexon-Kaliumkanal Partikel werden mit dissoziierten Hepatocyten in Kontakt gebracht. Verfahren zur Gewinnung von dissoziierten Hepatocyten sind dem Fachmann bekannt. Da Hepatocyten ebenfalls das Connexin Cx32 enthalten, lagern sie sich an die Partikel an und bilden mit ihnen Gap Junctions aus. Die Gap Junctions öffnen sich und bilden eine leitende Verbindung zwischen den Hepatocyten und den Partikel. Dies wird nachgewiesen, indem ein Farbstoff, z.B. Lucifer Yellow [13] in die Zellen hinein diffundiert, wenn die Partikel zuvor mit diesem Farbstoff beladen worden sind.

### Beispiel 3: Aktivitätsbestimmung eines nikotinergen Acetylcholinrezeptors

Es werden HEK Zellen mit cDNA zur Expression des Connexin 32 transfiziert und zugleich oder anschließend mit cDNA zur Expression eines ligandengesteuteren Ionenkanals, eines nikotinergen Acetylcholinrezeptors, transfiziert. Die Verfahren zur Herstellung derartiger Zellen und zur funktionellen Expression des Ionenkanals, sind allgemein bekannt und gehören zum Fachwissen des Durchschnittsfachmanns.

Zellen, welche beide Proteine exprimieren, werden nun mit den Partikeln aus Beispiel 1 in Kontakt gebracht. Sie lagern sich an die Partikel an und bilden mit diesen Gap Junctions aus, da sowohl die Partikel als auch die Zellen das Connexin 32 besitzen.

Die Aktivierung des nikotinergen Acetylcholinrezeptors mit einem geeigneten Agonisten, wie z.B. Nikotin, führt zu einer Änderung des Membranpotentials. Diese wird über die Gap Junctions auf die Membran des Partikels übertragen und wird optisch nachgewiesen werden. Hierfür werden die Partikel zuvor mit einem spannungssensitiven membranlöslichen Farbstoff (z.B. DIBAC [14]) beladen.

### Beispiel 4: Second messenger gekoppelte Rezeptoren

Zellen, die einen second messenger gekoppelten Rezeptor besitzen, werden zusätzlich mit einem Connexin transfiziert, so dass sie dieses Connexin zusätzlich zu den nativ vorhandenen Membranproteinen in ihrer Membran ausbilden. Sie werden mit Partikeln aus Beispiel 1 in Kontakt gebracht, die mit einem dazu kompatiblen Connexin hergestellt worden sind. Die Zellen lagern sich an die Partikel an. Durch die Gap Junctions wird eine Verbindung zwischen den Zellen und dem Innern der Partikel hergestellt. Werden nun die second messenger gekoppelten Rezeptoren in den Zellen aktiviert, so diffundieren die intrazellulären Reaktionsprodukte auch in die Partikel hinein und können dort durch eine entsprechende chemische Reaktion nachgewiesen werden. Zum Beispiel könnte dies der Nachweis einer erhöhten Calciumkonzentration durch FURA-2 sein, wie es in der Literatur an vielen Stellen als Nachweisverfahren ausführlich dokumentiert ist.

### Beispiel 5: Experimente an dissoziierten Herzzellen

Dissoziierte Herzzellen besitzen nativ das Connexin Cx43 [15]. Deshalb können sie sich an Partikel aus Beispiel 1 anlagern, die unter Verwendung des Connexin Cx43 hergestellt worden sind. Werden diese Zellen nun mit Wirkstoffen behandelt, die in den Herzzellen vorhandene Rezeptoren aktivieren, dann können die Effekte beispielsweise mit den in Beispiel 3 und 4 angegebenen Verfahren nachgewiesen werden.

### Literatur

[1] Austin, C.D. (1993) The Connexins: A Family of Gap Junction Proteins. Einstein Quarterly Journal of Biology and Medicine 10, 133-142
[2] Goodenough, D.A., J.A. Goliger, and D.L.Paul (1996) Connexins, Connexons, and intercellular communication. Annu. Rev. Biochem. 65:475-502
[3] Jordan K., Solan J.L., Dominguez M., Sia M., Hand A., Lampe P. and Laird D.W. (1999) Trafficking, Assembly, and Function of a Connexin43-Green Fluorescent Protein Chimera in Live Mammalian Cells. Molecular Biology ofthe Cell 10, 2033-3050
[4] Mazet et al. (1992) Voltage Dependence of liver gap-junction channels reconstituted into liposomes and incorporated into planar bilayers. European Journal of Biochemistry 210, 249-256
[5] Brewer (1991) Reconstitution of lens channels between two membranes. Chapter 19 in: Biophysics of Gap Junction Channels, Editor: C. Peracchia, CRC Press Boca Raton, Ann Arbor, Boston
[6] Phelan (2000) Gap Junction Communication in Invertebrates: The Innexin Gene Family. Current Topics in Membranes 49, 389-422
[7] Knight et al., A functional assay for G-protein-coupled receptors using stably transformed insect tissue culture cell lines. Anal Biochem. 2003. 320(1):88-103
[8] Hanke, W. (1985) Reconstitution of Ion Channels. CRC Critical Reviews Biochemistry 19, 1-44
[9] Sackmann E. and Tanaka M. (2000) Supported Membranes on soft polymer cushions: fabrication, characterization and applications TIBTECH 18, 58-64
[10] Loidl-Stahlhofen et al. (2001) Solid-Supported Biomolecules on Modified Silica Surfaces - A Tool for Fast Physicochemical Characterization and High-Throughput Screening. Advanced Materials 13, 1829-1834
[11] Raguse et al. (1998). Tethered Lipid Bilayer Membranes: Formation, and Ionic Reservoir Characterization. Langmuir 14, 648-659
[12] Hamill, O.P., A. Marty, E. Neher, B. Sakmann and F.J. Sigworth (1981). Improved patchclamp techniques for high-resolution current recording from cells and cell-free membrane patches. Pflügers Archiv 391, 85-100
[13] Cao et al. 1998. Journal Cell. Sci., 111, 31-43
[14] Whiteaker et al. 2001. Journal of Biomolecular Screening 6, 305-312
[15] Yeager et al. 1998. Current Opinion Struct. Biol. 8, 517-524

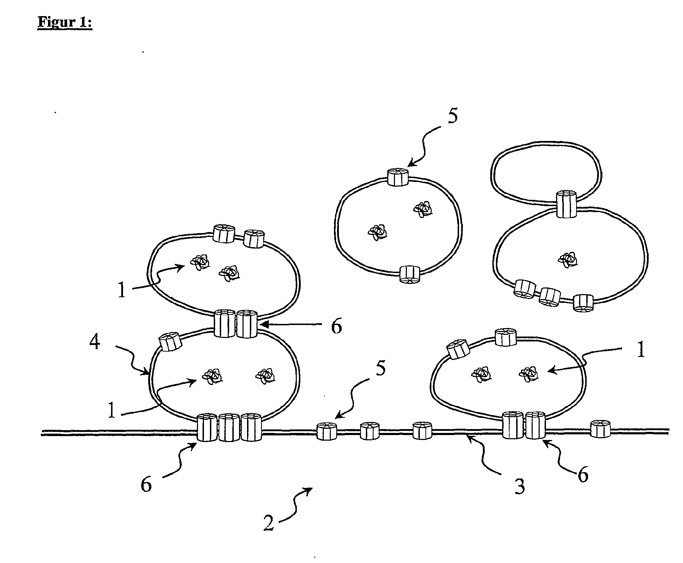

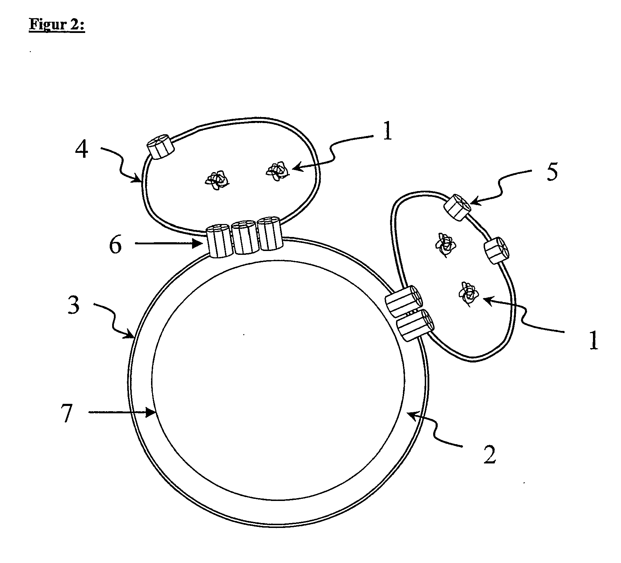

## Patentansprüche

1. Messanordnung zum Nachweis einer biochemischen Aktivität mit einer Nachweisreaktion für besagte biochemische Aktivität, **gekennzeichnet durch** einen Ort der biochemischen Aktivität und einen Ort der Nachweisreaktion, wobei der Ort der biochemischen Aktivität und der Ort der Nachweisreaktion **durch** mindestens zwei Lipidmembranen getrennt sind und mit Gap Junctions verbunden sind und wobei weiterhin der Ort der Nachweisreaktion ein künstlich erzeugter Membrankörper ist.

2. Messanordnung nach Anspruch 1, wobei besagte biochemische Aktivität in einer lebenden Zelle erfolgt.

3. Messanordnung nach einem der Ansprüche 1 bis 2, wobei die Nachweisreaktion eine Reaktion mit einem Farbstoff ist.

4. Messanordnung nach einem der Ansprüche 1 bis 2, wobei die Nachweisreaktion eine Reaktion mit einem Calcium-sensitiven Indikator ist.

5. Messanordnung nach einem der Ansprüche 1 bis 2, wobei die Nachweisreaktion eine Reaktion mit einem Spannungs-sensitiven Indikator ist.

6. Messanordnung nach einem der Ansprüche 1 bis 2, wobei die Nachweisreaktion eine Reaktion mit einem pH-Wert-sensitiver Indikator ist.

7. Messanordnung nach einem der Ansprüche 1 bis 6, wobei eine der Lipidmembranen als supported-bilayer ausgeführt ist.

8. Messanordnung nach Anspruch 7, wobei die supported bilayer als planare supported bilayer ausgeführt ist.

9. Messanordnung nach Anspruch 7, wobei die supported bilayer als sphärische supported bilayer ausgeführt ist.

10. Messanordnung nach Anspruch 7, wobei die supported bilayer auf einem sphärischen Silica-Träger aufgebracht ist.

11. Messanordnung nach einem der Ansprüche 1 bis 6, wobei mindestens eine Lipidmembran das Ende einer Kapillare überspannt.

12. Vorrichtung zur Bestimmung von biochemischen Aktivitäten enthaltend eine Vielzahl von Messanordnungen gemäß einem der Ansprüche 1 bis 11.

13. Verfahren zum Nachweis einer biochemischen Aktivität mit einer Nachweisreaktion, **gekennzeichnet durch** einen Ort der biochemischen Aktivität und einen Ort der Nachweisreaktion, wobei der Ort der biochemischen Aktivität und der Ort der Nachweisreaktion **durch** mindestens zwei Lipidmembranen getrennt sind und mit Gap Junctions verbunden sind und wobei weiterhin der Ort der Nachweisreaktion ein künstlich erzeugter Membrankörper ist.

14. Verfahren nach Anspruch 13, wobei besagte biochemische Aktivität eine Reaktion auf das Vorhandensein eines Wirkstoffs darstellt.

15. Verwendung einer Messanordnung nach einem der Ansprüche 1 bis 11 oder einer Vorrichtung nach Anspruch 12 zum Wirkstoff-Screening.

## Claims

1. Test set-up for detecting a biochemical activity using a detection reaction for the said biochemical activity, **characterized by** a site of the biochemical activity and a site of the detection reaction, wherein the site of the biochemical activity and the site of the detection reaction are separated by at least two lipid membranes and are connected by gap junctions and wherein, furthermore, the site of the detection reaction is an artificially produced membrane body.

2. Test set-up according to Claim 1, wherein the said biochemical activity takes place in a living cell.

3. Test set-up according to one of Claims 1 to 2, wherein the detection reaction is a reaction with a dye.

4. Test set-up according to one of Claims 1 to 2, wherein the detection reaction is a reaction with a calcium-sensitive indicator.

5. Test set-up according to one of Claims 1 to 2, wherein the detection reaction is a reaction with a voltage-sensitive indicator.

6. Test set-up according to one of Claims 1 to 2, wherein the detection reaction is a reaction with a pH-sensitive indicator.

7. Test set-up according to one of Claims 1 to 6, wherein one of the lipid membranes is in the form of a supported bilayer.

8. Test set-up according to Claim 7, wherein the supported bilayer is in the form of a planar supported bilayer.

9. Test set-up according to Claim 7, wherein the supported bilayer is in the form of a spherical supported bilayer.

10. Test set-up according to Claim 7, wherein the supported bilayer is applied to a spherical silica support.

11. Test set-up according to one of Claims 1 to 6, wherein at least one lipid membrane spans the end of a capillary.

12. Device for determining biochemical activities, which device contains a multiplicity of test set-ups according to one of Claims 1 to 11.

13. Method for detecting a biochemical activity using a detection reaction, **characterized by** a site of the biochemical activity and a site of the detection reaction, wherein the site of the biochemical activity and the site of the detection reaction are separated by at least two lipid membranes and are connected by gap junctions and wherein, furthermore, the site of the detection reaction is an artificially produced membrane body.

14. Method according to Claim 13, wherein the said biochemical activity constitutes a reaction to the presence of an active compound.

15. Use of a test set-up according to one of Claims 1 to 11 or of a device according to Claim 12 for active compound screening.

## Revendications

1. Dispositif de mesure pour la détection d'une activité biochimique par une réaction de détection pour ladite activité biochimique, **caractérisé par** un site d'activité biochimique et un site de réaction de détection, où le site de l'activité biochimique et le site de la réaction de détection sont séparés par au moins deux membranes lipidiques et sont reliés par des jonctions communicantes (gap junctions) et où d'autre part, le site de la réaction de détection est un corps membranaire produit de manière synthétique.

2. Dispositif de mesure selon la revendication 1, où ladite activité biochimique est réalisée dans une cellule vivante.

3. Dispositif de mesure selon les revendications 1 à 2, où la réaction de détection est une réaction avec un colorant.

4. Dispositif de mesure selon les revendications 1 à 2, où la réaction de détection est une réaction avec un indicateur sensible au calcium.

5. Dispositif de mesure selon les revendications 1 à 2, où la réaction de détection est une réaction avec un indicateur sensible à une tension.

6. Dispositif de mesure selon les revendications 1 à 2, où la réaction de détection est une réaction avec un indicateur sensible au pH.

7. Dispositif de mesure selon les revendications 1 à 6, où une des membranes lipidiques est exécutée comme une bicouche supportée.

8. Dispositif de mesure selon la revendication 7, où la bicouche supportée est exécutée comme une bicouche supportée plane.

9. Dispositif de mesure selon la revendication 7, où la bicouche supportée est exécutée comme une bicouche supportée sphérique.

10. Dispositif de mesure selon la revendication 7, où la bicouche supportée est appliquée sur un support sphérique en silice.

11. Dispositif de mesure selon les revendications 1 à 6, où au moins une membrane lipidique recouvre l'extrémité d'un capillaire.

12. Dispositif pour la détermination d'activités biochimiques, contenant un certain nombre de dispositifs de mesure selon l'une des revendications 1 à 11.

13. Procédé pour la détection d'une activité biochimique par une réaction de détection, **caractérisé par** un site d'activité biochimique et un site de réaction de détection, où le site de l'activité biochimique et le site de la réaction de détection sont séparés par au moins deux membranes lipidiques et sont reliés par des jonctions communicantes (gap junctions) et où d'autre part, le site de la réaction de détection est un corps membranaire produit de manière synthétique.

14. Procédé selon la revendication 13, où ladite activité biochimique est une réaction sur la présence d'un agent actif.

15. Utilisation d'un dispositif de mesure selon l'une des revendications 1 à 11 ou d'un dispositif selon la revendication 12, pour le criblage d'agents actifs.
